# EUROPEAN PATENT SPECIFICATION

(11) **EP 2 464 666 B1**
(45) Date of publication and mention of the grant of the patent: **21.03.2018**
(21) Application number: 10746994.2
(22) Date of filing: 14.07.2010
(51) Int. Cl.: C07K 16/24, C07K 16/28, A61P 35/00

(54) **COMBINATION OF A HUMAN INTERLEUKIN-6 ANTAGONIST AND A HUMAN INTERLEUKIN-6 RECEPTOR ANTAGONIST IN THERAPY OF TUMOUR DISEASES**
KOMBINATION AUS ANTAGONISTEN DES MENSCHLISCHEN INTERLEUKIN-6- UND ANTAGONISTEN DES MENSCHLISCHEN INTERLEUKIN-6-REZEPTORS ZUR BEHANDLUNG VON KREBS
COMBINAISON D'UN ANTAGONISTE DE L'INTERLEUKIN 6 HUMAINE ET D'UNANTAGONISTE DU RÉCEPTEUR HUMAIN DE L'INTERLEUKIN 6 POUR LE TRAITEMENT DES MALADIES TUMORALES

(30) Priority: 15.07.2009 CH 11082009
(43) Date of publication of application: 20.06.2012
(73) Proprietor: Kovacs-Benke, Eva, 4127 Birsfelden (CH)
(72) Inventor: Kovacs-Benke, Eva, 4127 Birsfelden (CH)
(86) International application number: PCT/CH2010/000183
(87) International publication number: WO 2011/006273

(56) References cited:
- WO-A1-2007/076927
- US-A- 6 086 874
- VAN ZAANEN H C T ET AL: "Endogenous interleukin 6 production in multiple myeloma patients treated with himeric monoclonal anti-IL6 antibodies indicates the existence of a positive feed-back loop" JOURNAL OF CLINICAL INVESTIGATION, vol. 98, no. 6, 1 September 1996 (1996-09-01), pages 1441-1448, XP008092204 AMERICAN SOCIETY FOR CLINICAL INVESTIGATION, US ISSN: 0021-9738 DOI: 10.1172/JCI118932
- KOVACS EVA: "Multiple myeloma and B cell lymphoma. Investigation of IL-6, IL-6 receptor antagonist (IL-6RA), and GP130 antagonist (GP130A) using various parameters in an in vitro model." THESCIENTIFICWORLDJOURNAL, vol. 6, 2006, pages 888-898, XP008127870 ISSN: 1537-744X
- MIMA TORU ET AL: "Clinical value of blocking IL-6 receptor." CURRENT OPINION IN RHEUMATOLOGY, vol. 21, no. 3, May 2009 (2009-05), pages 224-230, XP008127885 ISSN: 1531-6963
- KOVACS EVA: "Interleukin-6 leads to interleukin-10 production in several human multiple myeloma cell lines. Does interleukin-10 enhance the proliferation of these cells?" LEUKEMIA RESEARCH, vol. 34, no. 7, July 2010 (2010-07), pages 912-916, XP002604659 ISSN: 1873-5835
- GOUGELET ANGÉLIQUE ET AL: "Lymphoma and myeloma cell resistance to cytotoxic agents and ionizing radiations is not affected by exposure to anti-IL-6 antibody." PLOS ONE, vol. 4, no. 11, E8026, 2009, pages 1-13, XP002605105 ISSN: 1932-6203
- ULRIKE KLEIN ET AL: "Effective prophylaxis of thromboembolic complications with low molecular weight heparin in relapsed multiple myeloma patients treated with lenalidomide and dexamethasone", ANNALS OF HEMATOLOGY, SPRINGER, BERLIN, DE, vol. 88, no. 1, 31 July 2008 (2008-07-31), pages 67-71, XP019655015, ISSN: 1432-0584
- KOVACS-BENKE EVA: "Additive/Synergistic effects of Interleukin-6 and Interleukin-10 on the proliferation of human myeloma cells", INTERNATIONAL JOURNAL OF HEMATOLOGY RESEARCH, vol. 1, no. 3, October 2015 (2015-10), pages 74-78,

## Description

### Technical field

The prevent invention relates to an anti-tumour agent in combination of an anti-human Interleukin-6 monoclonal antibody and an anti-human Interleukin-6 receptor monoclonal antibody.

### Background of the invention

**Cytokines** are soluble proteins, peptides or glycoproteins that are released by cells. Cytokines can affect the cells via an autocrine and/or paracrine regulation mechanisms. In case of an autocrine regulation mechanism the endogenous produced cytokine affects the same type of cell. In case of a paracrine regulation mechanism the target cell is near to the cytokine produced cell. Cytokine binds to a specific receptor and causes a change in function or in development (differentiation) of the target cell. In both cases, i.e. autocrine and paracrine regulation mechanisms the expression of the membrane receptor is altered.

**Interleukins** are a group of cytokines which are produced by a wide variety of body cells. The majority of interleukins are synthesized by helper CD4+ T lymphocytes, monocytes/macrophages and by endothelial cells. If the produced cytokine is released, then it is measurable in the supernatant, if not then the cytokine is measurable only intracellular.

**Interleukin-6** (IL-6) originally defined as a B cell differentiation factor is produced by monocytes/macrophages, fibroblasts, endothelial cells, keratinocytes, B and T cells, mast cells, polymorphonuclear neutrophils, some nerve cells, muscle cells, adipocytes and certain tumour cells. Interleukin-6 acts as a pro-inflammatory and an anti-inflammatory cytokine. As a pro-inflammatory cytokine regulates inflammatory reactions either directly or indirectly. As an anti-inflammatory cytokine Interleukin-6 reduces the inflammatory reactions. IL-6 exits in three molecular weights, i.e. 21-30 kDa, 150-200 or 450 kDa. The biological activity of IL-6 depends on binding to its specific receptors. These membrane receptors composed the glycoprotein gp80 Interleukin-6 receptor alpha (IL-6R, also called CD126) and a signal-transducing component gp130 (also called CD 130). The complex IL-6+IL-6R+gp130 initiate a signal transduction cascade through JAKs (Janus kinases) and STATs (Signal Transducer-Activator of Transcription). The membrane receptors are released from the cells as soluble receptor proteins (sIL-6R and sgp130). As agonist, sIL-6R enhances the biological activity of IL-6 and sgp130 is an antagonist against the complex IL-6+sIL-6R.

**Disorders associated with abnormal Interleukin-6 production.** Dysregulated production of IL-6 is implicated in the pathogenesis of many diseases, such as, autoimmune diseases, chronic rheumatoid arthritis, Castleman's disease, inflammatory bowel disease, ulcerative colitis.

**Tumour diseases associated with abnormal Interleukin-6 production.** Several reports have described an association of elevated serum levels of Interleukin-6 in patients having different types of malignant tumours and with haematological malignant disorders: Colorectal cancer, endometrial cancer, renal cell carcinoma, non-small cell lung carcinoma, pancreatic cancer, breast and ovarian cancer : Yokoe et al., Breast Cancer 7: 187-190, 2000*;* Zhuang and Adachi., Anticancer Res 19: 1427-1432, 1999*;* Barber et al., Clin Sci 96: 83-87, 1991*;* Belluco et al., Ann Surg Oncol 7: 133-138, 2000*;* Hoheisel et al., Clin Invest 65:183-186, 1998*.*

In some patients Interleukin-6 could arise from immunocompetent cells and/or from malignant cells and/or from inflammatory infiltrates involved in the malignant process. How might the presence of IL-6 contribute to a poor prognosis for some cancer? *One possibility*: IL-6 is a growth factor for tumour cells. *Second possibility:* IL-6 suppresses the anti-tumour activity.

**Multiple myeloma (MM)** is a haematological disorders of clonal malignant plasma cells, that accounts for 1-2 % of all human cancer, the annual incidence is 3-6 per 100 000 people. Multiple myeloma (plasmozytoma) is characterised by slow proliferation of the tumour cells, mainly in the bone marrow, by production of large amounts of immunoglobulins and osteolytic lesions. Many cytokines are involved in the growth and survival of these tumour cells. Multiple myeloma is at present an incurable disease, but remissions may be induced with stem cells transplants, steroids, chemotherapy and treatment with thalidomide + dexamethasone or bortezomib based regimens or lenalidomide + dexamethasone (Klein et al., Annals of Hematology 88:67-71, 2008). the different therapeutic modalities have different "target location".

**Interleukin-6** is a major proliferative factor for the malignant plasma cells (multiple myeloma cells). This cytokine produces by the plasma cells and it affects the cells by an autocrine regulation mechanism with an additional paracrine signalling. IL-6 in a concentration of 2 pg/ml can induce 50% proliferation in myeloma cell lines.

Interleukin-6 enhances survival of the myeloma cells because it inhibits apoptosis of induction of the anti-Fas (Hata et al., Blood 86: 1039-1945, 1995)*.* (Nordan and Potter, Science 233: 566-569, 1986*).* The multiple myeloma cells can be classified into three groups depending on exogenous IL-6: (a) both proliferation and survival of the cells are dependent on IL-6, (b) only proliferation of the myeloma cells is affected by IL-6, (c) the cells are dependent on IL-6 only for survival, but not for proliferation. However there are also some cell lines that are independent of IL-6 both for survival and proliferation *(*Nilsson et al.; Mechanisms of B cell Neoplasie 15th Workshop, Basel, 1998*).* The serum values of IL-6 in 35% or in 97% or in 42% of multiple myeloma patients were significantly higher than in healthy persons *(*DuVillard et al., Brit J Haematol 89:243-249, 1995*;* Wierzbowska et al., Brit J Haematol 105: 412-419, 1999*;* Nachbour et al., Ann Hematol 62:54-58, 1991*).* Increased serum levels of IL-6 were found in patients with lymphoma *(*DuVillard et al., Brit J Haematol 89: 243-249, 1995*;* Yee et al., Blood 74:798-804, 1989*;* Stasi et al., Eur J Haematol 54; 9-17, 1995*;* Seymour et al., Am J Med 102; 21-28, 1997*;* Kato et al., Leuk Lymph 29; 71-79, 1997*).*

Because about 70% of the secreted IL-6 forms a complex with sIL-6R (Gaillard et al., Eur J Immunol 27: 332-3340, 1987), the amount of the free IL-6 in serum is low. The serum level of the sIL-6R is an important parameter in the evaluation and in the progression of multiple myeloma (Papadaki et al., Acta Haematologica 97: 191-195, 1997*;* Pulkki et al., Brit J Haematol 92: 370-374, 1996*;* Wierzbowska et al., Brit J Haematol 105: 412-419, 1999*).* The serum values of sIL-6R also were significantly increased in lymphoma patients and lymphoid malignancies *(*Lavabre-Bertrand et al., Brit J Haematol 91: 871-877, 1995*);* Ohtani et al., Brit JHaematol 91:116 120, 1995*).*

High sIL-6R levels have been reported in multiple myeloma in inflammatory bowel disease and in various malignant diseases *(*Mitsuyama et al., Gut 36: 45-49, 1995*;* Hoheisel et al., Clin Invest 65: 183-186, 1998*;* Jablonska, Cytokine 10: 540-543, 1998*;* Kovacs, Biomed Pharmacother 55: 391-396, 2001*).*

**Interleukin-10 (IL-10)** is known as a human cytokine synthesis inhibitory factor (CSIF). It produces by Thelper2 cells, monocytes/macrophages, by B lymphocytes and some tumour cells. Interleukin-10 has (1) immunosuppressive effect and (2) immunostimulatory effect. It down-regulates the expression of Thelper1 cytokines, MHC class II antigens and co stimulatory molecules on macrophages.

**Tumour diseases associated with abnormal Interleukin-10 production.** Several reports have described an association of elevated serum IL-10 levels in melanoma, ovarian cancer, gastrointestinal carcinoma, hepato-cellular carcinoma, lung cancer, renal carcinoma (Moore et al., Annu Rev Immunol 19: 683-765, 2001*;* Krüger et al., Br J Cancer 70: 1182-1185, 1994*;* Gotlieb et al., Cytokine 4: 385-390, 1992*;* De Vita et al., Cancer 86, 1936-1943, 1999*;* Chau et al., Ann Surg 231, 552-558, 2000*;* De Vita et al., Chest 117. 365-373, 2000*;* Wittke et al., Br J Cancer 79, 1182-1184*).*

How might the presence of IL-10 contribute to a poor prognosis for some cancer? *One possibility*: Interleukin-10 is a growth factor for tumour cells. *Second possibility*: Interleukin-10 suppresses the anti-tumour immune responses. Interleukin-10 enhances the survival and proliferation of B cells and can act as a cooperative factor in the proliferation of non-Hodgkin's lymphoma and of B cell lymphoma *(*Masood et al., Blood 85: 3423-3430, 1995*;* Jones et al., Blood 94: 2871-2879, 1999*;* Voorzanger et al., Cancer Research 56: 5499-5505, 1996*).* IL-10 is a growth factor for myeloma cells *(*Kovacs, Leukemia Research 34: 912-916, 2010*),* enhances the proliferation of freshly explanted myeloma cells in a short-term bone marrow culture *(*Lu et al., Blood 85: 2521-2527, 1995*).* Three out of seven human myeloma cell lines produce IL-10. Elevated IL-10 levels were detected in serum from about 50% of patients having multiple myeloma showing a relation to the clinical manifestation *(*Otsuki et al., Brit J Haematol 111: 835-842, 2000*;* Otsuki et al., Leuk Lymphoma 43: 969-974, 2002*).* Interleukin-6 leads to a marked production of Interleukin-10 in several human multiple myeloma cells. Interleukin-10 is an Interleukin-6 related growth factor for these tumour cells *(*Kovacs, Leukemia Research 34: 912-916, 2010*).*

### Detailed description of invention

The present invention relates a therapeutic drug contains the effective amount of the combination of an anti-human IL-6 monoclonal antibody (IL-6A) and an anti-human IL-6 receptor monoclonal antibody (IL-6RA).

An anti-human IL-6 monoclonal antibody is an antibody that specifically inhibits the biological activity of human IL-6 by bonding with IL-6.

An anti-human IL-6 receptor monoclonal antibody is an antibody that specifically inhibits the biological activity of human IL-6 receptor by bonding with IL-6 receptor.

### The following examples illustrate the invention.

### Cell lines and culture medium

Ten human multiple myeloma cell lines: RPMI-8226, OPM-2, U-266, LP-1, Molp-8, Molp-2, Colo-677, KMS-12BM, KMS-12-PE, Amo-1, were obtained from DSMZ (Braunschweig, Germany). Six tumour cell lines RPMI-8226, OPM-2, U-266, Molp-8, Molp-2 and LP-1 were derived from blood, Colo-677 from lymph node, KMS-12-BM from bone marrow, KMS-12-PE from peritoneum and Amo-1 from ascitic fluid.

The doubling times of tumour cell lines were between 35 and 96 hours. To measure parameters the cell cultures were used within 4-6 weeks after thawing. Culture medium: RPMI 1640 supplemented with 10-20% foetal calf serum, 2mM L-glutamine and 1 % gentamicin in a humidified atmosphere with 5% C0₂ at 37°C.

### Test substances

Recombinant human **Interleukin-6** (IL-6) and recombinant human **Interleukin-10** (IL-10) were obtained from R&D Systems (No 206-IL and No 1064-IL, United Kingdom) and reconstituted in phosphate-buffered saline with 0.18 % bovine serum albumin. The IL-6 was calibrated with the NIBSC/WHO international standard (1 µg IL-6 is equivalent to 1.1x10⁵ IU). Human **interleukin-6 receptor antagonist** (IL-6RA) was obtained from MedSystems, (No BMS 135, Diagnostics GmbH, Austria) and reconstituted in phosphate buffered saline (PBS). **Human gp130 antagonist** (gp130A) was obtained from PharMingen (No 555755; BD Biosciences, Germany) and reconstituted in PBS.

### Statistical analysis

For the evaluation of the parameters the Mann-Whitney U-test was used. The limit of significance was taken as P<0.05. For the correlation between the effects of the various test substances the Spearmann rank correlation test was applied. The limit of significance was taken as P<0.05.

### Brief description of the drawings

**Table 1** presents in ten human multiple myeloma cell lines (a) the spontaneous IL-10 production, (b) the IL-10 production induced by IL-6 and (c) the spontaneous IL-10 production inhibited by IL-6RA.
**Table 2** presents the IL-10 production in three human multiple myeloma cell lines in absence or in presence of IL-6, IL-6RA, IL-6+IL-6RA, gp130A and IL-6+gp130A.
**Table 3** presents the membrane expression of IL-6R and gp130 in three human myeloma cell lines in absence or in presence of IL-6, IL-6RA, gp130A.
**Table 4** presents the values of IL-10 and TNF-alpha production in three human multiple myeloma cell lines absence or in presence of IL-6, IL-6RA, IL-6+IL-6RA.
**Table 5** presents the proliferative effect of IL-6 and IL-10 in ten human multiple myeloma cell lines.
**Table 6** presents the effect of IL-6 and IL-6RA on the DNA synthesis in the human multiple myeloma cell line RPMI-8226.
**Figure 1** presents the expression of the membrane IL-6R and the membrane protein gp130 in myeloma cell line RPMI-8226 in absence or in presence of IL-6, IL-6RA, IL-6+IL-6RA, gp130A and IL-6+gp130A.
**Figure 2** presents the amount of IL-6 in supernatant after treatment with IL-6 or IL-6+IL-6RA in cell lines OPM-2 and RPMI-8226.
**Figure 3** presents the proliferation of cell lines OPM-2, RPMI-8226 and U-266 in absence or in presence of IL-6, IL-6RA and gp130A.

### EXAMPLE 1.

### Viability of multiple myeloma cells.

The ten human cell lines were cultivated at a density of 0.2-0.7x10⁶ cells/ml without the test substances (control) or with the test substances: (1) interleukin-6 (IL-6) (dose: 5 ng/10⁶ cells/ml); (2) interleukin-6 receptor antagonist (IL-6RA) (dose: 0.2 µg/10⁶ cells/ml); (3) gp130 antagonist (gp130A) (dose: 10 µg/10⁶ cells/ml); (4) Interleukin-10 (IL-10) (dose: 20 ng/10⁶ cells/ml) for 24 and 48 hours. The viabilities of the cells were determined by 7-amino-actinomycin D (7-AAD) to exclude the non-viable cells in flow cytometric assays. The viability of control cells without test substances lay in the range of 71-95 %. None of the substances impaired the viability of the cells.

### EXAMPLE 2.

### Interleukin-10 production in multiple myeloma cells (Table 1, Table 2).

Ten human multiple myeloma cell lines were cultivated for 24 hours and 48 hours at a density of 0.2-0.7x10⁶ cells/ml without test substances (spontaneous cytokine production) or with the test substances (induced cytokine production): (1) interleukin-6 (IL-6) (dose: 5 ng/10⁶ cells/ml); (2) interleukin-6 receptor antagonist (IL-6RA) (dose: 0.2 µg/10⁶ cells/ml); (3) IL-6+IL-6RA (dose: 5 ng/10⁶ cells/ml + 0.2 µg/10⁶ cells/ml); (4) gp130 antagonist (gp130A) (dose of 10 µg/10⁶ cells/ml); (5) IL-6+gp130A (dose: 5 ng/10⁶ cells/ml + 10 µg/10⁶ cells/ml). The treatment with gp130A and the combined treatments with IL-6+IL-6RA and IL-6+gp130A were carried out only in three tumour cell lines (RPMI-8226, OPM-2 and U-266) and only for 24 hours. IL-6RA and gp130A were added 2 hours before IL-6.

The interleukin-10 (IL-10) production in the supernatant of the cell cultures was determined by chemiluminescent immunometric assay. The lowest detectable level was 5 pg/ml.

**Table 1** shows the **IL-10** production in pg/ml at 24 and 48 h after treatment (range of 3-8 independent measurements). Spontaneous IL-10 production was found only in 4/10 cell lines: RPMI-8226, LP-1, Molp-8 and Colo-677. The two last cell lines (Molp-8 and Colo-677) secreted IL-10 not every time. **Interleukin-6** led to a marked increase of IL-10 production (up to 1626 pg/ml) in **6/10 cell lines.** The 6 cell lines included those cell lines which have spontaneous IL-10 production. In two tumour cell lines (RPMI-8226 and Molp-8) the IL-10 production was high during the two days, in the other four cell lines the production decreased slightly at 48 hours.

With IL-6RA the values were in the range of untreated samples.

**Table 2** shows the IL-10 production in cell lines RPMI-8226, OPM-2 and U-266. The cells were treated with IL-6, IL-6RA, IL-6+IL-6RA, gp130A and IL-6+gp130A for 24 hours. The measurements were repeated three or four times. RPMI-8226 produced IL-10 spontaneously, OPM and U-266 did not. IL-6 increased the IL-10 production in the three cell lines (P<0.05 and P<0.01). IL-6RA decreased the spontaneous IL-10 production of the RPMI-8226 significantly (P<0.05), for OPM-2 and U-266 the values were not detectable. With IL-6+IL-6RA the values were lower than with IL-6 (P<0.05 and P<0.01), but higher than with IL-6RA (P<0.01). Gp130A did not affect the spontaneous IL-10 production. After treatment with IL-6+gp130A the values were the same that with IL-6 in each case.

### EXAMPLE 3.

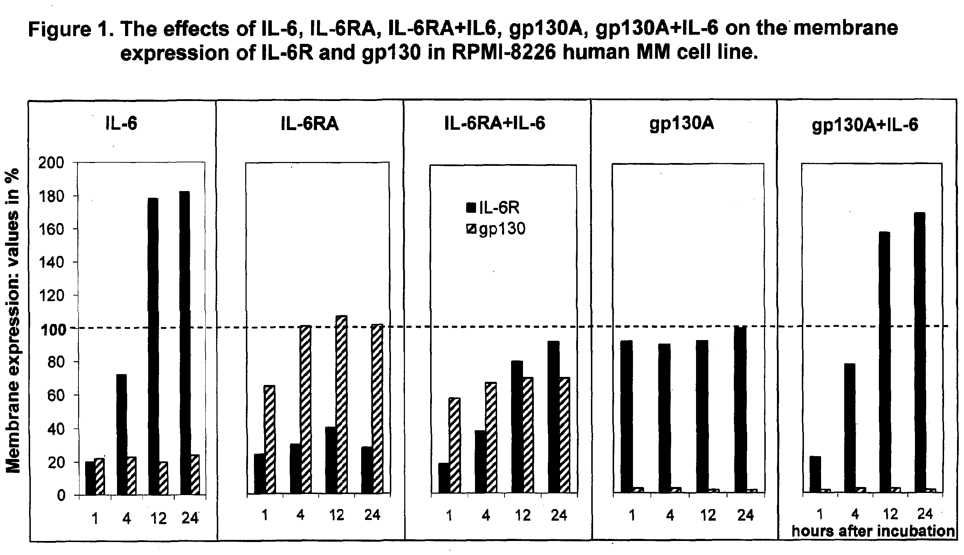

### Membrane expression of surface receptors in multiple myeloma cell lines (Table 3, Figure 1).

Three human multiple myeloma cell lines (OPM-2, RPMI-8226, U-266) were cultivated at a density of 0.2-0.7x10⁶ cells/ml for 24 hours with (1) IL-6 (dose: 5 ng/10⁶ cells/ml), (2) IL-6RA) (dose: 0.2 µg/10⁶ cells/ml), (3) IL-6+IL-6RA (dose: 5 ng/10⁶ cells/ml + 0.2 µg/10⁶ cells/ml), (4) gp130A (dose of 10 µg/10⁶ cells/ml, (5) IL-6+gp130A (5 ng/10⁶ cells/ml + 10 µg/10⁶ cells/ml). The combined treatment was carried out only in one tumour cell line (RPMI-8226) and measured 1, 4, 12 and 24 hours after treatment. IL-6RA or gp130A was added 2 hours before IL-6 at each time point. The membrane expression of IL-6 receptor and the membrane protein gp130 were measured using monoclonal antibodies (CD126 and CD130) in a FACS Calibur flow cytometer. For the expression of the membrane IL-6R and gp130 the signal intensity (geometric mean of the fluorescence intensity x counts) was used as parameter. The signal intensity of the treated samples was compared with that of untreated samples, which were taken as 100 %.

**Tables 3** present the signal intensities of surface Interleukin-6 receptor and gp130 in three myeloma cell lines. The values of four independent experiments are presented as mean±SEM.

### 1) Expression of Interleukin-6 receptor

The expressions of the membrane IL-6 receptor (IL-6R) were in the similar range in each cell line. Exogenous IL-6 significantly upregulated the membrane expression of its receptors in all multiple myeloma cell lines (range 133-186 %), (P<0.05 and P<0.01). IL-6 receptor antagonist (IL-6RA) inhibited the membrane receptor expression down to 51-83 % (P<0.05 and P<0.01). Higher doses of IL-6RA (0.4, 0.8, 1 and 2 µg/10⁶ cells) did not alter the receptor expression (results are not presented). Gp130 antagonist (gp130A) did not affect the surface IL-6R in any cell lines.

### 2) Expression of gp130 receptor

All three multiple myeloma cell lines expressed surface gp130. The membrane expressions were markedly different in the untreated multiple myeloma cell lines. Exogenous IL-6 downregulated the expression of surface protein gp130 down to 4-26% (P<0.01). IL-6RA did not affect membrane expression in any case. Gp130A inhibited the expression of surface gp130 completely (0.1-0.5%) in all cell lines.

**Figure 1** presents the kinetics of the membrane expression of IL-6R and gp130 after treatment with IL-6, IL-6RA, IL-6+IL-6RA, gp130A and IL-6+gp130A in multiple myeloma cell line RPMI-8226. The parameters were investigated in two independent experiments at different times (1, 4, 12 and 24 h). With exogenous IL-6 the expression of IL-6R was markedly upregulated, in contrast to it gp130 was downregulated. IL-6RA decreased the IL-6R expression, the membrane expression of gp130 was not inhibited with the exception of 1 hour. With IL-6+IL-6RA the kinetics curve of IL-6R was similar, but the values were lower than with IL-6. The membrane expression of gp130 was decreased, however less than with IL-6. Gp130A did not impair the membrane IL-6R, but inhibited gp130 completely. With IL-6+gp130A the kinetics curve and the values of IL-6R were the same as those of IL-6. The expression of the membrane gp130 was inhibited completely.

### EXAMPLE 4.

### The TNF-alpha production of multiple myeloma cells (Table 4).

Three human multiple myeloma cell lines (RPMI-8226, OPM-2, U-266) were cultivated at a density of 0.2-0.7x10⁶ cells/ml for 1, 4, 12, 24 and 48 hours with (1) IL-6 (dose: 5 ng/10⁶ cells/ml); (2) IL-6RA (dose: 0.2 µg/10⁶ cells/ml); (3) IL-6+IL-6RA (dose: 5 ng/10⁶ cells/ml + 0.2 µg/10⁶ cells/ml). For the combined treatment IL-6RA was added 2 hours before IL-6 at each time point.

The TNF-alpha production of the cell cultures was determined by chemiluminescent immunometric assay. The lowest detectable level was 5 pg/ml.

**Table 4** presents the values of the spontaneous and induced production of TNF-alpha in comparison with the values of IL-10. The values are the mean of three independent measurements. All three tumour cell lines produces TNF-alpha spontaneously in range of 10-14 pg/ml. IL-6 led to an marked increase of the TNF-alpha production already at 1 hour after incubation without alteration during 4, 12, 24 and 48 hours. With IL-6RA the values were in the range of spontaneous production. With IL-6+IL-6RA the TNF-alpha production was the same as by IL-6 incubation. The results show: (a) IL-6 induces both the TNF-alpha and IL-10 production, (b) IL-6RA does not affect the induced TNF-alpha production, but decreased the induced IL-10 production. These findings mean that the increased **TNF-alpha** production is influenced only by **IL-6,** for the increased production of **IL-10 is the IL-6 /IL-6R** complex responsible.

### EXAMPLE 5.

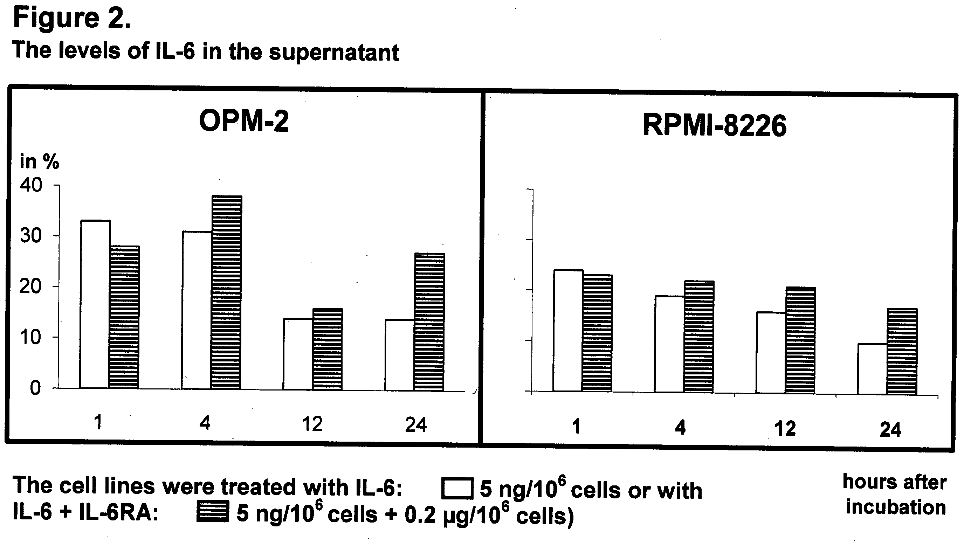

### The amount of IL-6 in the supernatant of multiple myeloma cells (Figure 2).

Two human multiple myeloma cell lines (OPM-2, RPMI-8226) were cultivated at a density of 0.2-0.7x10⁶ cells/ml for 1, 4, 12 and 24 hours with (1) IL-6 (dose: 5 ng/10⁶ cells/ml), 2) IL-6+IL-6RA (dose: 5 ng/10⁶ cells/ml + 0.2 µg/10⁶ cells/ml). IL-6RA was added 2 hours before IL-6 at each time point. The amount of IL-6 in the supernatant of the cell cultures was determined by chemiluminescent immunometric assay. The lowest detectable level was 2 pg/ml.

**Figure 2** presents the amount of IL-6 in the supernatant. The values are the mean of three independent measurements. After 24 hours the values were 14% (OPM-2) respectively 10% (RPMI-8226) those of the basis values. With the combined treatment (IL-6+IL-6RA) the amount of IL-6 in the supernatant was higher than without IL-6RA, after 24 hours the values lay were 27% (OPM-2) and 17 % (RPMI-8226).

The kinetics curve presents that the amount of IL-6 decreased during the 24 hours incubation time in both tumour cell lines.

### EXAMPLE 6.

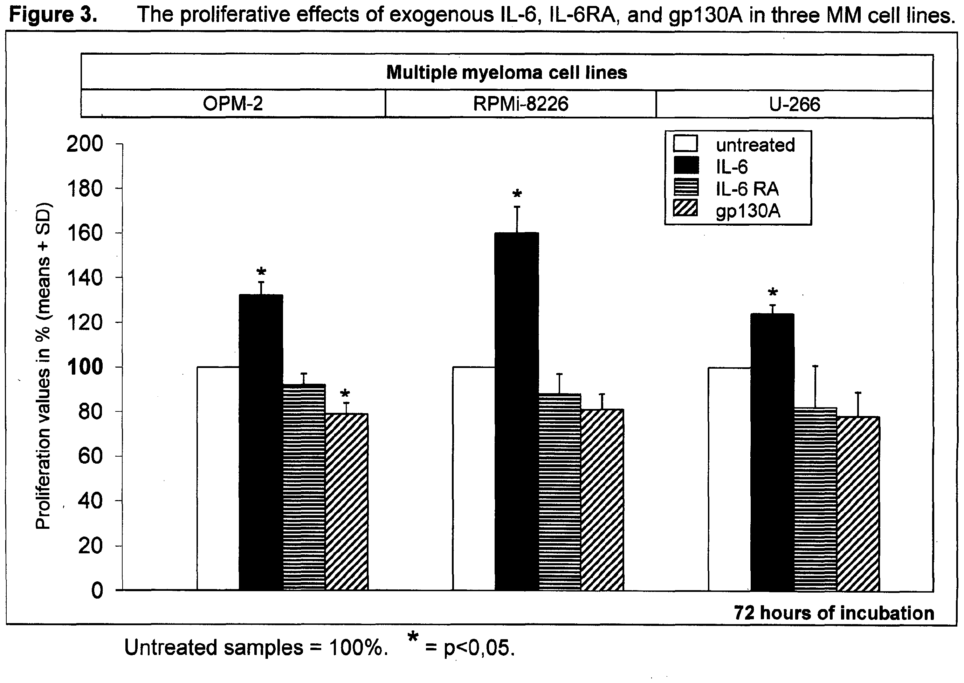

### The proliferation of multiple myeloma cells (Table 5, Table 6, Figure 3).

Ten human multiple myeloma cell lines (RPMI-8226, OPM-2, U-266, LP-1, Molp-8, Molp-2, Colo-677, KMS-12BM, KMS-12-PE, Amo-1) were investigated.

### 1) After incubation with Interleukin-6 or Interleukin-10

The cells were cultivated at a density of 0.2-0.7x10⁵ cells/100µl in 6-well plates. After 24 hours the cells were incubated for 24, 48 and 72 hours with IL-6 (dose: 0.5 ng/10⁵ cells/100µl) or IL-10 (dose: 2 ng/10⁵ cells/100 µl). The proliferation was measured at each time point using cell proliferation reagent WST-1. The mean values are expressed as percentage of the untreated samples (100%) and present the range of 4-7 independent measurements.

**Table 5** presents the proliferative effect of IL-6 and IL-10 in ten tumour cell lines. IL-6 led to an increase of proliferation in 9 out of 10 multiple myeloma cell lines with a rate of 110-160%. The proliferation was not altered in the cell line KMS-12 PE. With IL-10 the proliferation was increased in 7 out of 10 cell lines with a proliferation rate of 110-170%. IL-10 did not lead to an enhanced proliferation in the cell lines KMS-12-PE, Molp-8 and OPM-2. There was a significant positive correlation (P<0.05) between the proliferative effects of IL-6 and IL-10 in **6 out of 7** tumour cell lines.

### 2) After incubation with Interleukin-6receptor antagonist or gp130antagonist

The cells were treated with IL-6 (0.5 ng/10⁵ cells/100µl) for 72 hours or with IL-6RA (0.02 µg/10⁵ cells/100 µl) or gp130A (1µg/10⁵ cells/100 µl) for 24 hours after 48 hours of cultivation.

**Figure 3** shows the proliferative effects of exogenous IL-6, IL-6 RA and gp130A in three multiple myeloma cell lines (OPM-2, RPMI-8226, U-266). The values are expressed as percentage of the untreated samples and are the average of 3 or 4 independent experiments. IL-6 led to increases of proliferation in RPMI-8226 (up to a mean of 160%), in OPM-2 (up to a mean of 132%) and in U-266 (up to a mean of 124%) (P<0.05). When the surface IL-6 receptor was inhibited by its antagonist (IL-6RA) the spontaneous proliferation of the myeloma cells slightly decreased below that of the untreated samples (NS). Gp130A inhibited the spontaneous proliferation in all three myeloma cell lines (down to 81-78%). There was a significance (p<0.05) in OPM-2.

### 3) The DNA synthesis after incubation with Interleukin-6 or Interleukin-6 receptor antagonist

The DNA synthesis of the multiple myeloma cell line RPMI-8226 was measured by the incorporation of BrdU (5-bromo-2-deoxyuridine) in the proliferating cells. The cells were treated with IL-6 (0.5 ng/10⁵ cells/100µl) or with IL-6RA (0.02 µg/10⁵ cells/100µl) for 48 or 72 hours. IL-6RA was added 24 hours before measurement. **Table 6** presents the values of treated samples in comparison with untreated samples (100%). IL-6 led to increased values (up to 137 %). With IL-6RA the values lay in the range of untreated samples.

### DEFINITION

Monoclonal antibodies are monospecific antibodies that are identical because they are produced by one type of immune cells. These are clones of a single parent cell. Monoclonal antibodies could be used in cancer treatment that bind only to cell-specific antigens and induce an immunological response against the cancer cell.

Anti-human Interleukin-6 monoclonal antibody (IL-6A) binds specifically to Interleukin-6 leading to a high neutralising activity against the biological activity of human Interleukin-6. Anti-human Interleukin-6 receptor antibody (IL-6RA) binds specifically to membrane Interleukin-6 receptor and/or to the soluble Interleukin-6 receptor.

### Previous therapeutic modalities with Interleukin-6 antibody or Interleukin-6 receptor antibody.

Therapeutic targeting of IL-6 and its receptor in cancer has biologic rationale. Patients with B-lymphoproliferative disorders were treated with human anti-IL-6-antagonist successful (Haddad et al., Blood 97: 1590-1597, 2001)*.* Zaanen et al (Clin Invest 98: 1441-1448, 1996*)* reported that in multiple myeloma patients chimeric monoclonal anti-IL-6 antibodies strongly suppress endogenous IL-6 production. This IL-6 production is the 2-30 times higher in patients than in healthy persons. The anti-human IL-6R antibody as well as anti-human IL-6 antibody inhibits human myeloma growth in mice (Suzuki et al., Eur J Immunol 22: 1989-1993, 1992*).* An anti-human IL-6 monoclonal antibody in dose of 4 ng reduced the biological activity of 200 pg of human IL-6 by 50% *(*Nobuo et al., Patent Application No 90109583.6*).* Campo et al (Cytokine 31: 368-374, 2005*)* describe that the use of anti-IL-6 antibody did not improve the survival of human B cell lymphoma induced in mice. Voorhees et al (Clin Cancer Res 13: 6469-6478, 2007*; Patent, Application No* 20090022726*)* describe the use of a proteasome inhibitor (Bortezomib) in combination with an IL-6 antagonist (CNTO 328).

Anti-interleukin-6 receptor antibody Tocilizumab (RoActemra) as a humanised monoclonal antibody against IL-6R. It has been proposed this drug in the therapy of Castleman's disease and rheumatoid arthritis (Oldfield et al., Drugs 69: 609-632, 2009*).* Tocilizumab binds to sIL-6R and membrane IL-6R leading to the inhibition of the growth of the IL-6-dependent myeloma cell line KPMM2 (Mihara et al., Int Immunopharmacol 5: 1731-1740, 2005). Yoshida and Mizutan, Patent, Application 08/860,487) proposes the combination of IL-6A or IL-6RA or gp130A with different antiprolferative agents as mytomicin C or cisplatin or carboplatin in the treatment of renal carcinoma. Nishimoto et al (Patent Application 04792816.3) describe a therapeutic agent containing IL-6RA in the treatment of mesothelioma. Savino et al (Patent, Application No 20090035281*)* describe the use of the combination an IL-6RA, such as SANT7 and a glycocorticoid such as dexamethasone. SANT7 as an IL-6 receptor antagonist enhances the anti-tumour effect of dexamethasone (Tassone et al. Clin Cancer Res 11, 425-58, 2006*).*

### Disclosure of the invention

Interleukin-6 (IL-6), Interleukin-10 (IL-10), Interleukin-6 receptor antagonist (IL-6RA) and gp130 antagonist (gp130A)
The findings of the various studies are as followed:
(A) IL-6 or IL-10 or IL-6RA or gp130A do not impair the viability of the human multiple myeloma cells.
**(B)** IL-6 leads to a marked production of IL-10 in human multiple myeloma cell lines.
**(C)** IL-6 enhanced the proliferation of human multiple myeloma cells.
**(D)** IL-10 enhanced the proliferation of human multiple myeloma cells.
**(E)** IL-10 is an IL-6 related growth factor for human multiple myeloma cells.
**(F)** IL-6/IL-6R complex has a key position in the increased production of IL-10 in human multiple myeloma cells. It seems that gp130 is not necessary for the stimulation of the IL-10 production.
**(G)** TNF-alpha production of human multiple myeloma cells is influenced only by IL-6.
**(H)** IL-6RA decreased only slightly the spontaneous IL-10 production in human multiple myeloma cells (values are in the range of untreated samples).
**(I)** IL-6RA counteracted the increased IL-10 production induced by IL-6 treatment and decreased the high values of IL-10 significantly in human multiple myeloma cells.
**(J)** IL-6RA inhibits the spontaneous proliferation of these tumour cells slightly.
**(K)** Interleukin-10 is an Interleukin-6 related growth factor for human multiple myeloma cells *(*Kovacs, Leukemia Research 34, 912-916 2010*).*
**(L)** IL-6 affects the tumour cells via autocrine and/or paracrine regulation mechanisms. If the cells produce endogenous IL-6 and the cells express membrane IL-6 receptor the proliferation is regulated by autocrine way and paracrine way due to exogenous IL-6. If the cells do not produce endogenous IL-6, but express membrane IL-6R, the proliferation is regulated only by paracrine way.
(**M**) IL-6RA decreases the membrane expression of the IL-6R inhibiting the effects of endogenous IL-6 and exogenous IL-6. According to the findings of different investigations: (a) IL-6RA does not inhibit the membrane expression of the IL-6R completely (b) IL-6RA could be a non-competitive antagonist. Consequently: (1) the effects of endogenous or of exogenous IL-6 on the proliferation is not abolished efficiently (2) the available IL-6 bound to IL-6R leads to enhanced proliferation of the tumour cells and (3) the available IL-6 bound to IL-6R stimulates IL-10 production leading to an enhanced proliferation of the tumour cells.

**It is very important to inhibit the effect of IL-6 using the combination of a specific antibody to IL-6 and a specific antibody to IL-6R.**

**Table 1. The production of IL-10 in human multiple myeloma cell lines.**

| **Cell Line** | **Source** | **IL-10 production (pg/ml)** | | |
|---|---|---|---|---|
| | | **spontaneous** | **treated with** | |
| | | | **IL-6** | **IL-6RA** |
| **RPMI-8226** | | | | |
| | blood | **33-179** | **373-1626** | **28-160** |
| **OPM-2** | | | | |
| | blood | ND | **15-78** | ND |
| **U-266** | | | | |
| | blood | ND | **13-20** | ND |
| **LP-1** | | | | |
| | blood | **9-28** | **28-155** | **7-23** |
| **Molp-8** | | | | |
| | blood | ND-4 | **251-1217** | ND |
| **Molp-2** | | | | |
| | blood | ND | ND | ND |
| **Colo-677** | | | | |
| | lymph node | ND-**38** | **14-132** | ND |
| **KMS-12-BM** | | | | |
| | bonemarrow | ND | ND | ND |
| **KMS-12-PE** | | | | |
| | pleura | ND | ND | ND |
| **Amo-1** | | | | |
| | ascites | ND | ND | ND |

| | | | | |
|---|---|---|---|---|
| ND = not detectable | | | | |

**Table 2. The effects of IL-6, IL-6 receptor antagonist (IL-6RA) gp130 antagonist (gp130A) on the IL-10 production (pg/ml).**

| Treatment | **Untreated** | **IL-6** | **IL-6RA** | **IL-6 + IL-6RA** | **gp130A** | **IL-6 + gp130A** |
|---|---|---|---|---|---|---|
| Cell line | | | | | | |
| RPMI-8226 | 73 ± 15 | 939 ± 98 | 48 ± 14 | 380 ± 22 | 67 ± 18 | 849 ± 75 |
| OPM-2 | <5 | 45 ± 10 | <5 | 15 ± 8 | <5 | 48 ± 13 |
| U-266 | <5 | 19 ± 4 | <5 | 10 ± 3 | <5 | 17 ± 3 |

**Table 3. The effects of IL-6, interleukin-6 receptor antagonist (IL-6RA) and gp130 antagonist (gp130A) on the expression of the surface IL-6 receptor and membrane protein gp130 in human multiple myeloma cell lines.**

| Treatment | untreated samples | | IL-6 (5 ng /10⁶ cells | | IL-6 RA 0.2 µg/10⁶ cells | | gp130A 10 | µg/10⁶ cells |
|---|---|---|---|---|---|---|---|---|
| Cell line | IL-6 R | gp130 | IL-6 R | gp130 | IL-6 R | gp130 | IL-6 R | gp130 |
| OPM-2 | 568'305 ± 24'717 | 101'212 ± 3'187 | 927'146 ± 27'037^{a} | 4'267 ± 913^{b} | 402'553 ± 24'802 | 100'003 ± 7'464 | 562'125 ± 20'100 | 304 ± 9 |
| | | | 163% | 4% | 71% | 99% | 99% | 0,3% |
| RPMI-8226 | 420'688 ± 85'446 | 588'311 ± 127'065 | 781'793 ± 177'667^{b} | 155'741 ± 31'329^{b} | 214'708 ± 41'872 | 585'523 ± 129'102 | 416'203 ± 80'012 | 2'941 ± 78 |
| | | | 186% | 26% | 51% | 99% | 99% | 0,5% |
| U-266 | 697'726 ± 20'383 | 114'308 ± 5'679 | 927'074 ± 46'350^{a} | 20'975 ± 1'541 ^{b} | 579'195 ± 9'491 | 107'700 ± 4'828 | 682'120 ± 170'502 | 114 ± 10 |
| | | | 133% | 17% | 83% | 95% | 98% | 0,1% |

| | | | | | | | | |
|---|---|---|---|---|---|---|---|---|
| a = p<0.05 b = p<0.01 compared with untreated samples, ND not detectable | | | | | | | | |

**Table 4.**

| The production of TNF- alpha (TNF-a) in human multiple myeloma cell lines in comparison with IL-10 production. | | | | | | | | | | | | | |
|---|---|---|---|---|---|---|---|---|---|---|---|---|---|
| Time | Zytokine | RPMI-8226 | | | | OPM-2 | | | | U-266 | | | |
| | | **Co** | **IL-6** | **IL-6RA** | **IL-6** + **IL-6RA** | **Co** | **IL-6** | **IL-6RA** | **IL-6** + **IL-6RA** | **Co** | **IL-6** | **IL-6RA** | **IL-6 + IL-6RA** |
| 1 h | TNF-α IL-10 | 10 | 90 | 11 | 97 | 12 | 111 | 12 | 104 | 10 | 98 | 11 | 99 |
| | | 23 | 19 | 13 | 15 | <5 | <5 | <5 | <5 | <5 | <5 | <5 | <5 |
| 4 h | TNF-α IL-10 | 11 | 116 | 11 | 97 | 11 | 102 | 12 | 111 | 12 | 108 | 9 | 118 |
| | | 44 | 104 | 12 | 37 | <5 | 8 | <5 | <5 | <5 | <5 | <5 | <5 |
| 12 h | TNF-α IL-10 | 12 | 98 | 10 | 95 | 13 | 133 | 13 | 117 | 13 | 116 | 13 | 112 |
| | | 78 | 685 | 63 | 205 | <5 | 25 | <5 | 18 | <5 | 16 | <5 | 9 |
| 24 h | TNF-α IL-10 | 14 | 114 | 14 | 129 | 12 | 118 | 12 | 106 | 13 | 117 | 11 | 106 |
| | | 90 | 1171 | 70 | 489 | <5 | 42 | <5 | 20 | <5 | 17 | <5 | 12 |
| 48 h | TNF-α IL-10 | 13 | 101 | 13 | 120 | **NO MEASUREMENTS** | | | | 14 | 109 | 12 | 103 |
| | | 137 | 1018 | 100 | 507 | | | | | <5 | 18 | <5 | 13 |

| | | | | | | | | | | | | | |
|---|---|---|---|---|---|---|---|---|---|---|---|---|---|
| Values in pg/ml | | | | | | | | | | | | | |

**Table 5. The proliferative effects of IL-6 and IL-10 in human multiple myeloma cell lines.**

| **Cell line** | **Source** | **Proliferation (%)** | | **Correlation** |
|---|---|---|---|---|
| | | **after IL-6** | **after IL-10** | **IL-6 versus IL-10** |
| **RPMI-8226** | | | | |
| | blood | **133-148** | **113-130** | p<0.05 |
| **OPM-2** | | | | |
| | blood | **115-129** | 100 | NS |
| **U-266** | | | | |
| | blood | **110-115** | **110-125** | NS |
| **LP-1** | | | | |
| | blood | **125-160** | **118-140** | p<0.05 |
| **Molp-8** | | | | |
| | blood | **122-158** | 100 | NS |
| **Molp-2** | | | | |
| | blood | **113-118** | **110-117** | p<0.05 |
| **Colo-677** | | | | |
| | lymph node | **120-150** | **121-170** | p<0.05 |
| **KMS-12-BM** | | | | |
| | bone marrow | **110-138** | **112-145** | p<0.05 |
| **KMS-12-PE** | | | | |
| | pleura | 100 | 100 | NS |
| **Amo-1** | | | | |
| | ascites | 118-127 | 117-122 | p<0.05 |

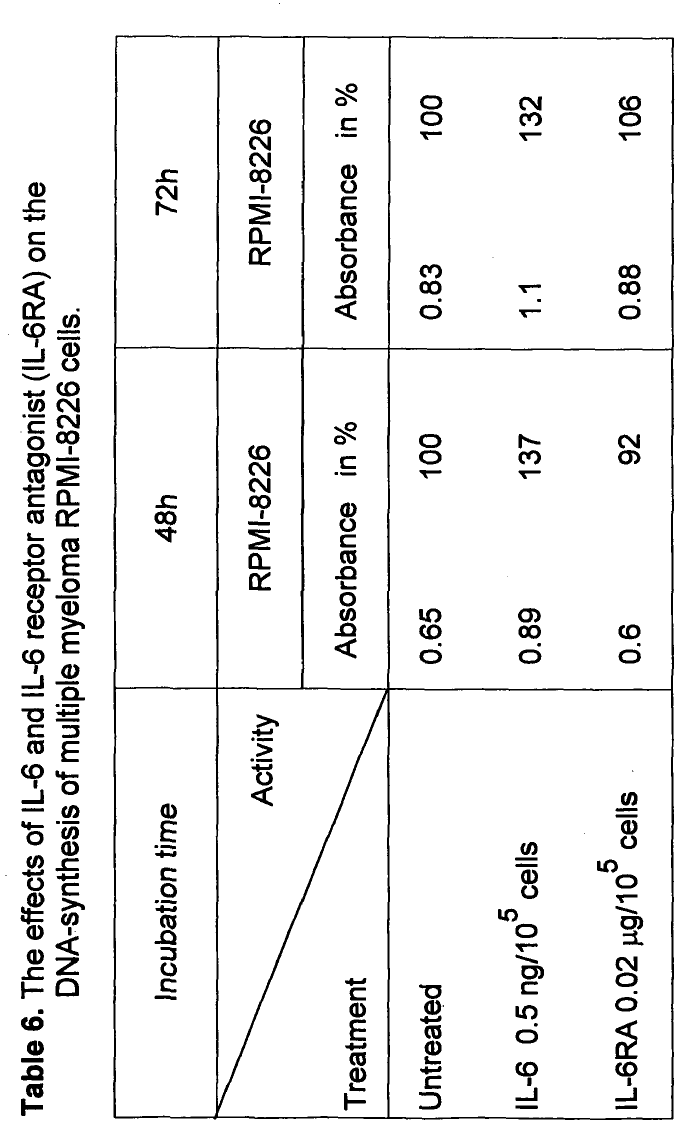

## Claims

1. The combination of an anti-human Interleukin-6 monoclonal antibody and an anti-human Interleukin-6 receptor monoclonal antibody for use in the treatment of human multiple myeloma in which exogenous or endogenous Interleukin-6 produces Interleukin-10 in myeloma cells leading to an enhanced proliferation of multiple myeloma (plasmocytoma).

2. The combination of an anti-human Interleukin-6 monoclonal antibody and an anti-human Interleukin-6 receptor monoclonal antibody for use in the treatment of Interleukin-6-dependent human multiple myeloma (plasmocytoma).

3. Combination for use according to claims 1 or 2, wherein anti-human Interleukin-6 monoclonal antibody is totally incapable of binding gp130**.**

4. Combination for use according to claims 1 or 2, wherein anti-human Interleukin-6 receptor monoclonal antibody is totally incapable of binding gp130**.**

5. The combination for use according to claims 1 or 2, wherein the monoclonal antibodies are administered per oral and/or parenteral.

## Patentansprüche

1. Die Kombination eines monoklonalen anti-human-Interleukin-6-Antikörpers und eines monoklonalen anti-human-Interleukin-6-Rezeptor-Antikörpers zur Verwendung für die Behandlung von human Multiplen Myelom, in dem exogenes oder endogenes Interleukin-6 in den Myelomzellen Interleukin-10 produziert und damit zu einer verstärkten Proliferation des Multiplen Myeloms (Plasmozytom) führt.

2. Die Kombination eines monoklonalen anti-human-Interleukin-6-Antikörpers und eines monoklonalen anti-human-Interleukin-6-Rezeptor-Antikörpers zur Verwendung für die Behandlung von Interleukin-6-abhängigem human Multiplen Myelom (Plasmozytom).

3. Die Kombination zur Verwendung gemäss Ansprüche 1 oder 2, bei denen der monoklonale anti-human-Interleukin-6-Antikörper grundlegend nicht fähig ist, sich an gp130 zu binden.

4. Die Kombination zur Verwendung gemäss Ansprüche 1 oder 2, bei denen der monoklonale anti-human-Interleukin-6-Rezeptor-Antikörper grundlegend nicht fähig ist, sich an gp130 zu binden.

5. Die Kombination zur Verwendung gemäß Ansprüche 1 oder 2, bei denen die monoklonalen Antikörper peroral und / oder parenteral verabreicht werden.

## Revendications

1. La combinaison entre un anticorps monoclonal anti-humain Interleukin-6 et d'un anticorps monoclonal anti-humain Interleukin-6 récepteur dans le cadre d'une utilisation pour le traitement d'un myélome humain multiple, procédé durant lequel de l'Interleukin-6 exogène ou endogène produit de Interleukin-10 au seins de cellules de myélome, donnant lieu à une prolifération augmentée de myélomes multiples (plasmocytome).

2. La combinaison d'un anticorps monoclonal anti-humain Interleukin-6 et d'un anticorps monoclonal anti-humain Interleukin-6 récepteur dans le cadre d'un traitement de myélomes multiples humains (plasmocytome) dépendants d'Inlerleukin-6.

3. La combinaison de l'emploi selon les revendications 1 et 2, où un anticorps monoclonal anti-humain Interleukin-6 est totalement incapable de lier du gp130.

4. La combinaison de l'emploi selon les revendications 1 et 2, où un anticorps monoclonal anti-humain Interleukin-6 récepteur est totalement incapable de lier du gp130.

5. La combinaison de l'emploi selon les revendications 1 et 2, où les anticorps monoclonaux sont administrés par voies orales et/ou parentérales.
